# EUROPEAN PATENT APPLICATION

(11) **EP 4 316 496 A1**
(43) Date of publication of application: **07.02.2024**
(21) Application number: 22773397.9
(22) Date of filing: 17.01.2022
(51) Int. Cl.: A61K 33/00, A23L 33/10, A61K 9/14, A61P 31/04, A61P 43/00

(54) **INTESTINAL BACTERIA COUNT REDUCING AGENT**

(30) Priority: 24.03.2021 JP 2021050445
(71) Applicant: OSAKA UNIVERSITY, Suita-shi Osaka 565-0871 (JP)
(72) Inventor: KAYAMA Hisako, Suita-shi, Osaka 565-0871 (JP); TAKEDA Kiyoshi, Suita-shi, Osaka 565-0871 (JP); KOBAYASHI Hikaru, Suita-shi, Osaka 565-0871 (JP); KOBAYASHI Yuki, Suita-shi, Osaka 565-0871 (JP)
(74) Representative: Schön, Christoph
(86) International application number: PCT/JP2022/001299
(87) International publication number: WO 2022/201775

(57) **Abstract**

An intestinal bacteria reducing agent of the present invention is a reducing agent which contains silicon particles or silicon fine particles and reduces the number of bacteria in the intestine. The intestinal bacteria reducing agent can reduce the number of bacteria in the intestine by a new action that cannot be explained by the heretofore known hydrogen generating capability of silicon particles or silicon fine particles, that is, the capability of eliminating hydroxyl radicals.

## Description

### TECHNICAL FIELD

The present invention relates to an intestinal bacteria inhibiting agent, a pharmaceutical product containing an intestinal bacteria reducing agent, and a food or beverage containing an intestinal bacteria reducing agent.

### BACKGROUND ART

In the midst of the growing health consciousness, about 70% of immune cells are said to exist in the intestine, and there is an increase in attention to the balance of a variety of intestinal bacteria which are called an intestinal flora and influence immune cells. However, due to a change in living environment including dietary life, stress or the like, which is faced by modern people, it is not as easy as before to regulate the balance of intestinal bacteria.

It is indicated that hydrogen generated as silicon fine particles orally ingested by an animal react with water in a certain environment in the body can eliminate active oxygen typified by hydroxyl radicals, and therefore silicon fine particles can be a prophylactic or therapeutic agent for some diseases (Patent Documents 1 to 12).

### PRIOR ART DOCUMENTS

### PATENT DOCUMENTS

Patent Document 1: International Publication WO 2019/021769
Patent Document 2: Japanese Patent Laid-open Publication No. 2019-214556
Patent Document 3: Japanese Patent Laid-open Publication No. 2020-7300
Patent Document 4: Japanese Patent Laid-open Publication No. 2020-79240
Patent Document 5: Japanese Patent Laid-open Publication No. 2020-79228
Patent Document 6: Japanese Patent Laid-open Publication No. 2020-117480
Patent Document 7: Japanese Patent Laid-open Publication No. 2020-117481
Patent Document 8: Japanese Patent Laid-open Publication No. 2020-117482
Patent Document 9: Japanese Patent Laid-open Publication No. 2020-117483
Patent Document 10: Japanese Patent Laid-open Publication No. 2020-117484
Patent Document 11: Japanese Patent Laid-open Publication No. 2020-117485
Patent Document 12: Japanese Patent Laid-open Publication No. 2020-117486

### NON-PATENT DOCUMENTS

Non-Patent Document 1: ANDOH, Factors influencing the gut microbiota structure, Modern Media, Vol. 65, 2019, 72-79
Non-Patent Document 2: Hydrogen-Rich Saline Regulates Intestinal Barrier Dysfunction, Dysbiosis, and Bacterial Translocation in a Murine Model of Sepsis, Mitsunori Ikeda et al., SHOCK Vol.50, No.6,2018,640-647

### SUMMARY OF THE INVENTION

### PROBLEMS TO BE SOLVED BY THE INVENTION

An object of the present invention is to provide an intestinal bacteria reducing agent containing silicon particles or silicon fine particles, which can form at least a part of a drug, food or beverage for regulating the intestinal environment, or a drug for various diseases associated with abnormality of intestinal bacteria.

### SOLUTIONS TO THE PROBLEMS

The present inventors examined the efficacy of silicon fine particles against an intestinal disease with animals (mice), and found that the number of intestinal bacteria and the abundance ratio of the bacteria, which were examined separately from the history of the disease, unexpectedly significantly changed before and after the ingestion of silicon particles or silicon fine particles described later. More specifically, the present inventors found that the number of intestinal bacteria in feces after the mice ingested the silicon particles or the silicon fine particles significantly decreased as compared to that before the ingestion, and the abundance ratio of the intestinal bacteria was significantly different from the abundance ratio when hydrogen water was ingested (cited from Non-Patent Document 2).

However, in view of the heretofore known hydrogen generating capability, that is, hydroxyl radical eliminating capability, which is developed particularly in a neutral or alkaline state by silicon fine particles, it is an unexpected result that the above-described events have been confirmed. This is because, as is seen from Non-Patent Document 1 published in 2019, the above-described finding is a result opposite to the common technical knowledge at least as of 2019.

Specifically, for example, with regard to anaerobic bacteria, Non-Patent Document 1 indicates that anaerobic bacteria "undergo DNA damage and protein damage by hydroxyl radicals and the like generated by reduction of oxygen because they do not have an enzyme which eliminates the hydroxyl radicals and the like". Therefore, Non-Patent Literature 1 suggests that for anaerobic bacteria, hydroxy radicals threat their survival. In other words, Non-Patent Document 1 suggests that in an environment where hydroxyl radicals are removed or reduced, at least the possibility that the number of anaerobic bacteria decreases is extremely low because an environment friendly to survival of anaerobic bacteria is formed.

However, as described above, when the present inventors caused normal animals (mice) or intestinal inflammation model animals (mice) to ingest silicon particles, silicon fine particles or aggregates thereof, and examined the number of bacteria in the intestine, a very interesting phenomenon was confirmed with good producibility in which the number of bacteria remarkably decreased regardless of whether aerobic or anaerobic. In addition, the present inventors further conducted studies and analysis on influences on bacteria in the intestine, and found that the abundance ratio of intestinal bacteria, particularly the abundance ratio of anaerobic bacteria, was significantly different from the abundance ratio when hydrogen water was ingested.

As a result, the present inventors have found that ingestion of silicon particles, silicon fine particles or aggregates thereof (hereinafter, also referred to collectively as "silicon fine particles") produces a new action that cannot be explained by the heretofore known hydrogen generating capability of the particles, that is, the capability of eliminating hydroxyl radicals, leading to construction of the present invention.

An intestinal bacteria reducing agent of the present invention is a reducing agent which contains silicon particles or silicon fine particles and reduces the number of bacteria in the intestine.

The intestinal bacteria reducing agent can reduce the number of bacteria in the intestine by a new action that cannot be explained by the heretofore known hydrogen generating capability of silicon fine particles, that is, the capability of eliminating hydroxyl radicals. Examples of the new action include actions of adsorbing, bonding and capturing intestinal bacteria and breaking cell membranes of intestinal bacteria by silicon fine particles, and an action of reduction by silicon fine particles, but the action is not limited thereto.

A pharmaceutical product containing the intestinal bacteria reducing agent is capable of changing the balance of bacteria in the intestine in addition to reducing the number of bacteria in the intestine. Examples of the disease targeted by the pharmaceutical product include obesity, diabetes, inflammatory bowel disease, appetite disorder, Parkinson's disease, Alzheimer's disease, multiple sclerosis, autism, allergy, arthritis, eczema, asthma, nonalcoholic hepatitis, atherosclerosis disease, hypertension, bacterial food poisoning, and multidrug-resistant bacterial infection.

A food or beverage containing the intestinal bacteria reducing agent is capable of changing the balance of bacteria in the intestine in addition to reducing the number of bacteria in the intestine. The type of the food or the beverage is not limited. For example, the food or the beverage is a preferred embodiment of health foods, foods with functional claims, foods for specified health uses and the like. The form of the food or the beverage is not limited. For example, a form of a mixture obtained by mixing the intestinal bacteria reducing agent with an existing food, or a formulated form of the intestinal bacteria reducing agent is an embodiment that can be employed. Examples of the formulated form include tablets, capsule preparations, powders, granules, and jellies.

The "silicon fine particles" in the present application include, as main particles, particles having an average crystallite diameter of 1 nm or more and less than 1 µm. In a narrower sense, the "silicon fine particles" in the present application include, as main particles, silicon nanoparticles having an average crystallite diameter at a nano level, specifically a crystalline diameter of 1 nm or more and 500 nm or less. In addition, the "silicon particles" in the present application include, as main particles, particles having an average crystallite diameter of more than 500 nm (in a narrow sense, 1 µm or more) and 500 µm or less.

In addition, in the present application, the "silicon fine particles" include not only those in which silicon fine particles are dispersed, but also those in which a plurality of silicon fine particles are aggregated to form aggregates in a µm order (generally 0.1 µm or more). In addition, the "silicon particles" may aggregate to form aggregates. Each of the above-described numerical value ranges for "silicon fine particles" is a typical example, and the numerical value range may in a more limited way. In addition, the crystallite diameter is appropriately selected depending on the application, use method, required function and the like of the "silicon fine particles" or the "silicon particles". In addition, the term "crystallite diameter" is employed as a term to refer to the size of crystallites of silicon fine particles, and the term "crystallite diameter" is distinguished from the term "aggregate diameter" which refers to the overall diameter of an aggregate of silicon particles. The particle size distribution of the aggregate diameter is not particularly limited.

In addition, the material of the "pH adjusting agent" (hereinafter, referred to as an "alkali agent") in the present application is not particularly limited as long as it is an agent capable of adjusting the pH value to fall within an alkali range of more than 7 (typically more than 7.4). Examples of the alkali agent include sodium hydrogencarbonate, sodium carbonate, sodium dihydrogen phosphate, disodium hydrogenphosphate, potassium hydrogencarbonate, potassium carbonate, and other pH adjusting agents for medicaments. Among them, sodium hydrogencarbonate is most widely used, and has a plurality of advantages such that it has a pH value adjustment function, and is excellent in safety and versatility. For any of the pH adjusting agents, a form in which the pH adjusting agent is not decomposed by an acid is a preferred aspect. In addition, when the intestinal bacteria reducing agent of the present application is orally ingested, a form is preferable in which the intestinal bacteria reducing agent is not decomposed or is hardly decomposed by gastric acid.

### EFFECTS OF THE INVENTION

An intestinal bacteria reducing agent of the present invention can reduce the number of bacteria in the intestine by a new action that cannot be explained by the heretofore known hydrogen generating capability of silicon particles or silicon fine particles, that is, the capability of eliminating hydroxyl radicals.

### BRIEF DESCRIPTION OF THE DRAWINGS

Fig. 1 shows implementation plans (protocols) for examining various indices of the number of intestinal bacteria under an intestinal bacteria reducing agent (feed) according to a first embodiment, in which a mouse model is used.
Fig. 2 shows bar graphs in which the numbers of respective types of living intestinal bacteria in feces when animals (mice) ingest the feed according to the present embodiment (Example 1) are compared with the numbers of respective types of living intestinal bacteria in feces when the animals ingest normal feed (comparative example).
Fig. 3 shows circular graphs in which the abundance ratios of respective types of intestinal bacteria in feces when animals (mice) ingest the feed according to the first embodiment (Example 2) are compared with the abundance ratios of respective types of intestinal bacteria in feces when the animals ingest normal feed (comparative example).
Fig. 4 shows a graph showing the relative numbers of representative Gram-negative bacteria when animals (mice) ingest the feed according to a first comparative embodiment (Example 2) and representative Gram-negative bacteria when the animals ingest normal feed (comparative example).
Fig. 5 shows circular graphs cited from Non-Patent Document 2, which show the abundance ratios of respective types of intestinal bacteria in feces before and after animals (mice) ingest hydrogen water.
Fig. 6 shows images showing the results of staining large intestine tissues of the animals (mice) in Example 2 and the comparative example by an immunohistostaining method.
Fig. 7 shows bar graphs in which the numbers of respective types of intestinal bacteria in feces when mice lacking a gene encoding IL-10, which are intestinal inflammation model animals (mice), ingests the feed according to the first embodiment (Example 3) are compared with the numbers of respective types of intestinal bacteria in feces when the animals ingest normal feed (comparative example).
Fig. 8 shows SEM (scanning electron microscope) photographs showing the result of observing silicon fine particles in the intestinal bacteria reducing agent and intestinal bacteria in Example 4 and the result of the comparative example.
Fig. 9 shows a further enlarged version of the SEM photograph showing the result of observing silicon fine particles in the intestinal bacteria reducing agent and intestinal bacteria in Example 4.
Fig. 10 shows a SEM photograph at 40,000-fold magnification, which shows the result of observing intestinal bacteria in the cecum in Example 5.
Fig. 11 shows another SEM photograph at 35,000-fold magnification, which shows the result of observing intestinal bacteria in the cecum in Example 5.

### EMBODIMENTS OF THE INVENTION

Embodiments of the present invention will be described on the basis of the accompanying drawings.

### <FIRST EMBODIMENT>

An intestinal bacteria reducing agent according to the present embodiment, and a method for producing the intestinal bacteria reducing agent are as follow.

### [1] Intestinal bacteria reducing agent

An example of the intestinal bacteria reducing agent according to the present embodiment is formed as a solid preparation by carrying out a step (kneading step) of kneading normal feed (manufactured by Oriental Yeast Co., Ltd., model number AIN 93M) as a base material, silicon fine particles capable of generating hydrogen particularly in a neutral or alkaline state by coming into contact with water, that is, silicon fine particles having a hydrogen generating capability, and an aqueous citric acid solution using a known kneading apparatus. As described later, the silicon fine particles of the present embodiment can have a hydrogen generating capability which enables reduction of the number of intestinal bacteria and/or a cell membrane breaking capability which enables formation of pores of in the cell membranes of bacteria.

### [2] Method for producing intestinal bacteria reducing agent

Next, a method for producing the intestinal bacteria reducing agent according to the present embodiment.

In the present embodiment, for example, silicon fine particle powder (e.g., particle size: 300 µm or less, purity: 4 N (i.e., 99.99% or more), i-type silicon) is used as a part of the intestinal bacteria reducing agent. In another aspect of the present embodiment, silicon fine particle powder having a purity higher than or lower than the aforementioned purity can be employed. The silicon fine particle powder according to the present embodiment is crystalline silicon powder, but even if the silicon fine particle powder is amorphous silicon or porous silicon, at least a part of the effects of the present embodiment can be exhibited.

### <Classification step>

First, in an example of the present embodiment, a classification step (first classification step) of classifying the silicon fine particle powder is carried out. Specifically, the first classification step is carried out using a sieve so that the ratio of the silicon fine particles having a crystallite diameter of 45 µm or more (including silicon fine particles or aggregates thereof as described above) to all the silicon fine particles is 5 mass% or less (more preferably 3 mass% or less, still more preferably 1 mass% or less, even more preferably 0.5 mass% or less, even more preferably 0.2 mass% or less). As a result, in the present embodiment, the silicon fine particles having a crystallite diameter of less than 45 µm are obtained, and substantially all of the silicon fine particles having a crystallite diameter of 45 µm or more are removed. (an example of process of production of silicon particles and silicon fine particles)

The upper limit or the lower limit of the crystallite diameter of the silicon fine particles according to the present embodiment is not limited as long as the silicon fine particles have a hydrogen generating capability particularly in a neutral or alkaline state. However, in addition to or in place of the first classification step, a classification step (second classification step) for removing substantially all of the silicon fine particles having a crystallite diameter of less than 1 µm may also be employed. (an example of process of production of silicon particles and silicon fine particles)

Here, the method for producing the intestinal bacteria reducing agent according to the present embodiment does not necessarily require the above-described first classification step and/or second classification step. However, carrying out the first classification step and/or second classification step is a preferred aspect from the viewpoint that a more stable hydrogen generating capability can be exhibited, the viewpoint that deterioration of human mouthfeel can be suppressed in the case of oral ingestion, and/or the viewpoint that the silicon fine particles can be reliably prevented from being directly absorbed to enter the blood vessel.

### <Pulverization step>

Next, a pulverization step is carried out in which the silicon fine particle powder obtained by passing through the classification step or without passing through the classification step is pulverized in a 99.5 wt% ethanol solution by a bead mill method using 0.5 mmϕ zirconia beads. As a result, the silicon fine particles obtained by passing through the classification step can more reliably give silicon fine particles (silicon nanoparticles) having a representative crystallite diameter of less than 100 nm. (a process of production of silicon fine particles)

An aspect in which instead of the pulverization step, another known pulverization method (roller mill method or the like) is used to obtain silicon fine particles (silicon nanoparticles) having a crystallite diameter of less than 500 nm is an aspect that can be employed. However, from the viewpoint of preventing metal contamination, employment of a bead mill method as a pulverization step is a preferred aspect. When the bead mill method is employed, the size and/or type of beads can be appropriately selected for obtaining silicon fine particles (including silicon fine particles) having a desired crystallite diameter.

By carrying out the above-described steps, the silicon fine particles (silicon particles, silicon fine particles or aggregates thereof) or the intestinal bacteria reducing agent according to the present embodiment can be produced. Therefore, the intestinal bacteria reducing agent as an example of the present embodiment can be produced without passing through a kneading step described later. Examples of the intestinal bacteria reducing agent include those obtained by incidentally or naturally molding the silicon fine particles according to the present embodiment with a physiologically acceptable pressure sensitive adhesive (including water; the same applies hereinafter), thickener or the like, and those intentionally molded with a physiologically acceptable pressure-sensitive adhesive, thickener or the like using a known tableting machine or the like. The shape of the silicon fine particles is not limited. For example, an indefinite shape, a polygonal shape, a sphere shape, an elliptical shape, a disk shape, a cylindrical shape or the like is an aspect of the silicon fine particles.

An example of the hydrogen generating capability of the silicon fine particles according to the present embodiment is as follows.

When silicon fine particles having a representative crystallite diameter of less than 500 nm and obtained by passing through the first classification step and the pulverization step are immersed in an aqueous solution (pH value: 8.2) containing sodium hydrogen carbonate as a solute and having a temperature equivalent to the human body temperature (37°C), the amount of generation of hydrogen until passage of 24 hours after the start of the immersion is about 300 mL/g.

### <Kneading step>

In an example of the present embodiment, an intestinal bacteria reducing agent of the example can be produced by normal feed as a base material, the silicon fine particles according to the present embodiment which have a hydrogen generating capability, and an aqueous citric acid solution using a known kneading apparatus as described above after the pulverization step.

The type of the base material is not limited as long as it is a physiologically acceptable material.

An example of the ratio (mass ratio) of the silicon fine particles to the normal feed is such that the amount of silicon fine particles is 2.5 when the amount of the normal feed is assumed to be 100. The amount of the aqueous citric acid solution is not particularly limited, and can be set to, for example, about 0.5 wt% with respect to the total amount of the silicon fine particles and the normal feed. In addition, the pH value of the aqueous citric acid solution is about 4.

The intestinal bacteria reducing agent according to the present embodiment contains citric acid together with the normal feed and silicon fine particles, but use of the above-described pH adjusting agent, that is, an alkaline agent, instead of the citric acid is another aspect that can be employed. However, from the viewpoint of reliably exhibiting at least the hydrogen generating capability of the silicon fine particles according to the present embodiment in the human or non-human intestine, ingestion of the intestinal bacteria reducing agent with the pH adjusting agent and the silicon fine particles filled and contained in a known enteric-coated capsule is a preferred aspect.

### <Molding step>

Using a commercially available pelleter to mold the intestinal bacteria reducing agent containing the silicon fine particles according to the present embodiment with consideration given to the suitability of a dosage form in ingestion is also an aspect that can be employed. After molding, moisture is removed using a dryer heated to about 90°C, and classification is performed using a sieve. In this way, an intestinal bacteria reducing agent as a solid preparation (feed) can be produced. From the viewpoint of preventing or inhibiting the intestinal bacteria reducing agent from coming into contact with moisture, the intestinal bacteria reducing agent being packaged in a known water-impermeable covering material and stored is a preferred aspect. In addition, the ratio (mass ratio) of the silicon fine particles to the normal feed is not limited. The amount of the representative silicon fine particles is 0.02 to 70 when the amount of the normal feed is assumed to be 100.

The citric acid contained in the intestinal bacteria reducing agent according to the present embodiment can serve to adjust the pH value of pure water to 4 or more and less than 7 (in a more limited sense, 6 or less) when the intestinal bacteria reducing agent is disintegrated in the pure water. As a result, the pH value adjustment action of citric acid that sets the water-containing liquid to an acidic state can prevent or inhibit the generation of hydrogen due to the intestinal bacteria reducing agent being brought into contact with moisture and the like in the outside air. Therefore, as one suitable aspect according to the present embodiment, the intestinal bacteria reducing agent contains the citric acid. Even when the intestinal bacteria reducing agent according to the present embodiment does not contain the citric acid, at least a part of the effects of the present embodiment can be exhibited.

At least a part of the surface of the silicon fine particle may include a film of silicon oxide (including silicon dioxide) which is naturally formed by exposure to air or moisture or artificially formed by a known film formation method (including hydrogen peroxide water treatment). The thickness of the film of silicon oxide is not limited as long as the silicon fine particles have a hydrogen generating capability.

### <Confirmation test for number of intestinal bacteria>

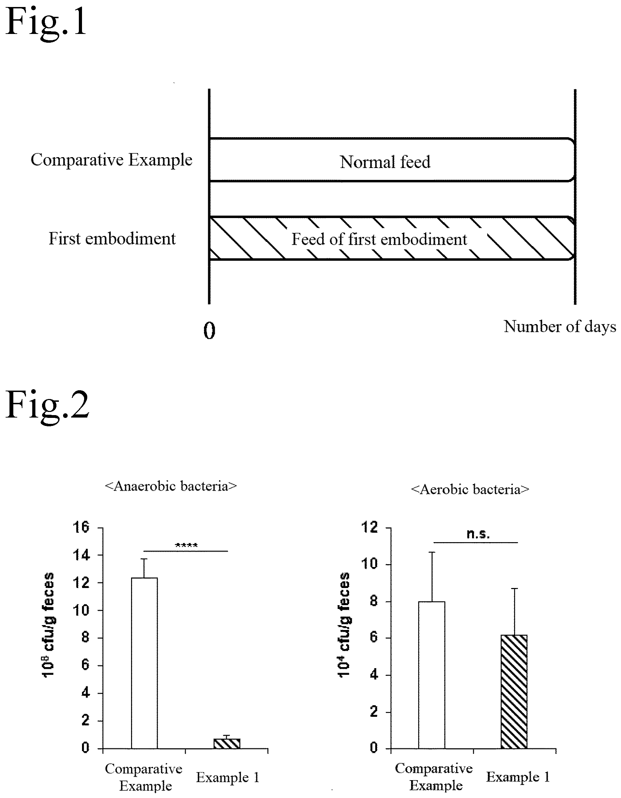

Fig. 1 shows implementation plans (protocols) for examining various indices of the number of intestinal bacteria under an intestinal bacteria reducing agent (feed) according to the present embodiment, in which C57BL/6J mice (hereinafter, referred to as "mice") acquired from Japan SLC, Inc. are used.

### (Example 1)

In the confirmation test of the present example, 7-week old normal C57BL/6J mice were employed.

The intestinal bacteria reducing agent produced in accordance with the above-described production method, in which the amount of the silicon fine particles is 2.5 when the amount of the normal feed is assumed to be 100 in terms of mass ratio, was used as feed for the mice (hereinafter, referred to as "feed of Example 1").

The "number of days" in Fig. 1 in the present example is 5. Therefore, in the present example, during 5 days before use of the mice in the confirmation test, the mice of an intestinal bacteria reducing agent administration group was allowed to freely take the feed of Example 1 and water, and a control group (comparative example) was allowed to freely take the normal feed (manufactured by Oriental Yeast Co., Ltd., model number AIN 93M) and water.

Fig. 2 shows bar graphs in which the numbers of respective types of living intestinal bacteria in feces when mice ingest the feed of Example 1 (Example 1) are compared with the numbers of respective types of living intestinal bacteria in feces when the mice ingest normal feed (comparative example). For the measurement of the number of intestinal bacteria, the present inventors analyzed the number of bacteria (colony forming unit/g (feces) (CFU)) in the feces of the mice (in other words, in the excrement) after the bacteria were cultured using a static culture method.

As shown in Fig. 2, it was confirmed that at least the number of anaerobic bacteria in the present example significantly decreased as compared to the number of bacteria in the comparative example. In particular, it is worth noting that the number of anaerobic bacteria in the present example decreases to 1/10 or less of the number of anaerobic bacteria in the comparative example.

### <Confirmation test for abundance ratio of intestinal bacteria>

### (Example 2)

In the present example, a confirmation test for examining an increase or decrease in the numbers of aerobic and anaerobic intestinal bacteria and abundance ratios of the intestinal bacteria was conducted. The "number of days" in Fig. 1 in the present example is 7. Therefore, in the present example, a confirmation test for examining an increase or decrease in the numbers of the respective intestinal bacteria and abundance ratios of the intestinal bacteria was conducted using the feed of Example 1 and the 7-week old mice employed in Example 1.

In the present example, during 7 days before use of the mice in the confirmation test, the mice of an intestinal bacteria reducing agent administration group was allowed to freely take the feed of Example 1 and water, and a control group (comparative example) was allowed to freely take the normal feed and water.

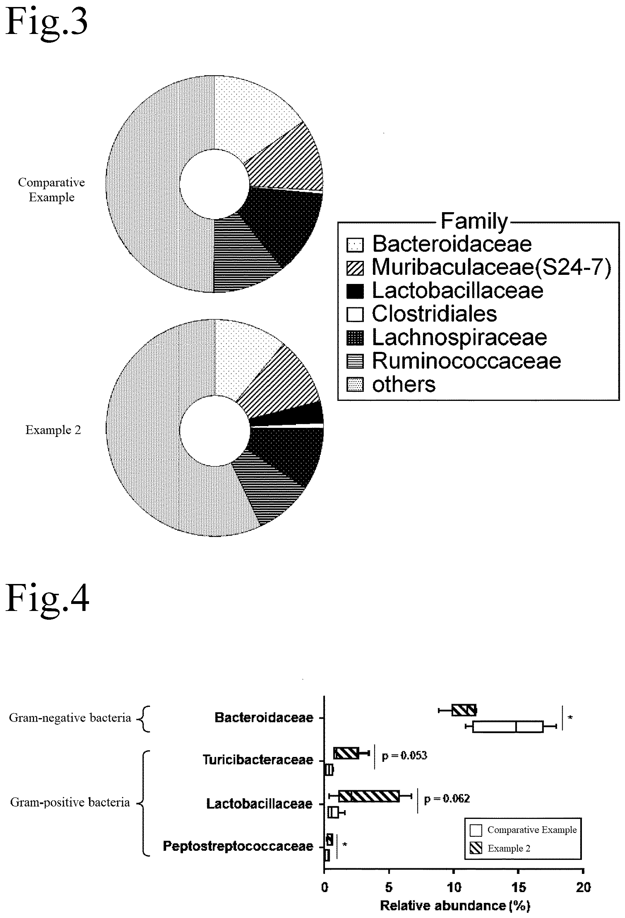

Fig. 3 shows circular graphs in which the abundance ratios of respective types of intestinal bacteria in feces when mice ingest the feed according to the present embodiment (Example 2) are compared with the abundance ratios of respective types of intestinal bacteria in feces when the mice ingest normal feed (comparative example).

The abundance ratios of intestinal bacteria in Example 2 and the comparative example were obtained by a method in which with deoxyribonucleic acid (DNA) collected from the feces in each of the examples, 16S rRNA genes of all bacteria are amplified by a polymerase chain reaction (PCR) method, and the nucleotide sequence of the variable region of each of 16S rRNA gene fragments contained in the PCR amplification product obtained by the amplification is then examined to identify the types of the intestinal bacteria. Therefore, the abundance ratios (%) of intestinal bacteria in Example 2 and the comparative example are each obtained by multiplying 100 by a value obtained using the total number of 16S rRNA gene fragments in the PCR amplification product as a denominator and the number of gene fragments the respective types of intestinal bacteria having a specific nucleotide sequence in the variable region as a numerator. In the present application, the above-described "total number of 16S rRNA gene fragments" is synonymous with "all bacteria". Specific examples of the abundance ratio (%) of the respective intestinal bacterium are as shown in Table 1 below.

**[Table 1]**

| Type of bacteria | Abundance ratio of respective bacteria of comparative example to all bacteria (%) | Abundance ratio of respective bacteria of Example 2 to all bacteria (%) |
|---|---|---|
| Bacteroidaceae (Anaerobic) | **15. 178** | **10. 848** |
| Muribaculaceae (Anaerobic) | **10. 794** | **10. 202** |
| Lactobacillaceae (Anaerobic) | **0. 504** | **3. 225** |
| Clostridiales (Anaerobic) | **0. 115** | **0. 867** |
| Lachnospiraceae (Anaerobic) | **12. 646** | **9. 226** |
| Ruminococcaceae (Anaerobic) | **10. 843** | **8. 634** |
| Others | **49.92** | **56. 998** |
| Total (%) | **100** | **100** |

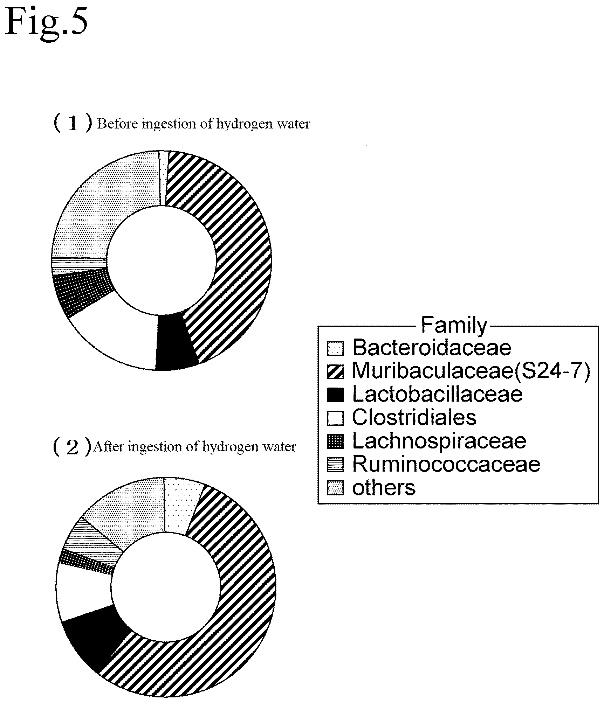

Fig. 4 shows a graph showing the relative ratios of the number of representative Gram-negative bacteria and the number of Gram-positive bacteria when mice ingest the feed according to the present embodiment (Example 2) and when the mice ingest normal feed (comparative example). Fig. 5 shows circular graphs (reference data) cited from Non-Patent Document 2, which show the abundance ratios of respective types of intestinal bacteria in feces before and after mice ingest hydrogen water.

As shown in Figs. 3 and 4, it was confirmed that the abundance ratio of anaerobic bacteria to all bacteria in at least Example 2 decreased significantly or remarkably in more aggressive phraseology as compared to the abundance ratio of bacteria in the comparative example, and the abundance ratios of the respective types of intestinal bacteria changed.

Very interestingly, the present inventors confirmed at least the following two characteristics ((a) and (b)) from Figs. 2 to 4 including Example 1.
(a) The number of Bacteroidaceae, gram-negative anaerobic bacteria that can be said to be harmful to humans, in Example 2 dramatically decreased as compared to that in the control group (comparative example).
(b) The abundance ratio of Bacteroidaceae in Example 2 to the total number of bacteria in the intestine is lower than the abundance ratio of Bacteroidaceae in the comparative example to the total number of bacteria in the intestine.

Therefore, it was confirmed that the intestinal bacteria reducing agent according to the present embodiment was characterized by reducing the ratio of the number of anaerobic bacteria (in particular, Gram-negative anaerobic bacteria) to the total number of at least the intestinal bacteria shown in Fig. 3.

Here, by observing the circular graphs disclosed in Non-Patent Document 2, which show the abundance ratios of respective types of intestinal bacteria in feces before and after mice ingest hydrogen water (Fig. 5), a difference between the results of ingestion of hydrogen water and the present example (Example 2) is more clearly understood.

Specifically, comparison between the data disclosed in Non-Patent Document 2 and Example 2 led to interesting findings that as shown in Fig. 5, the abundance ratio of anaerobic bacteria (particularly Gram-negative anaerobic bacteria, more particularly Bacteroidaceae) to all bacteria in the intestine decreases, whereas quite the contrary, the abundance ratio of the anaerobic bacteria to all the bacteria in the intestine increases when hydrogen water is ingested.

Further, in the present example, the large intestines of the mice were collected, and the host cell nucleus (DAPI), mucus (antimouse MUC2 antibody), and intestinal bacteria (eubacterial probe EUB 338) were stained using an immunohistochemical staining method.

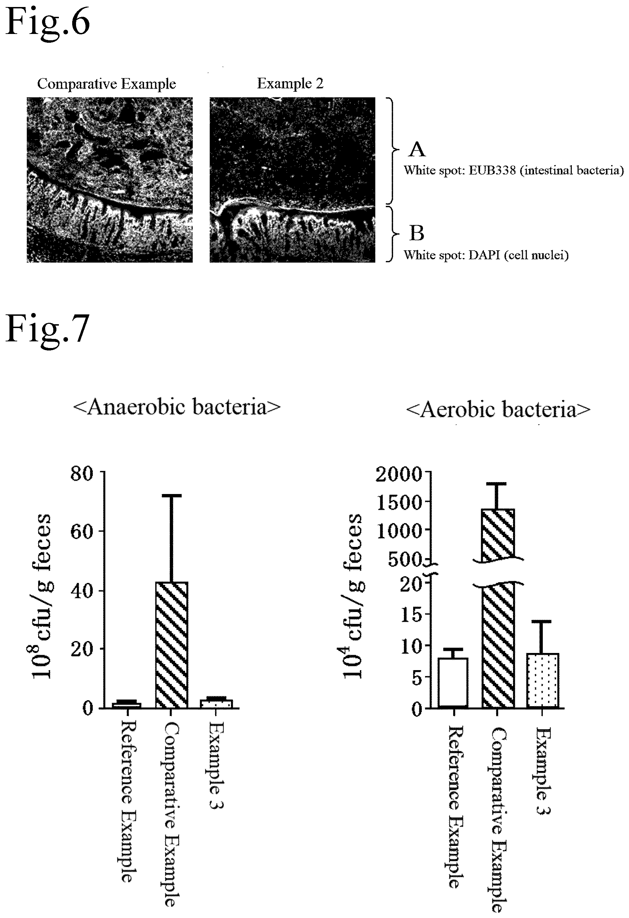

Fig. 6 shows images showing the results of staining large intestine tissues of the mice in Example 2 and the comparative example by an immunohistostaining method. The region "A" of Fig. 6 is a lumen region, and the region "B" of Fig. 6 is an epithelium and lamina propria region. White spots in the luminal region (region "A") represent intestinal bacteria, and white spots in the epithelium and lamina propria region (region "B") represent cell nuclei.

The results revealed that as shown in Fig. 6, the number of intestinal bacteria labeled with the EUB 338 probe decreased significantly or remarkably in more aggressive phraseology in the lumen of the mouse large intestine.

Therefore, also from Fig. 6, it is confirmed that the feed of Example 1 remarkably reduces the number of intestinal bacteria.

### <Confirmation test for number of intestinal bacteria (2)>

### (Example 3)

For examining the intestinal bacteria reducing effect in intestinal inflammation model animals that spontaneously develop intestinal bacteria-dependent intestinal inflammation by lacking a gene encoding anti-inflammatory cytokine IL-10 (hereinafter, "intestinal inflammation model animals"), a test was conducted in which the effect of continuously ingesting the feed of Example 1 over a period longer than the period in Example 1 (5 days) was confirmed using normal animals (mice) and intestinal inflammation model animals. The "number of days" in Fig. 1 in the present example is 161 (23 weeks). Therefore, in the present example, a confirmation test for examining a change in the number of intestinal bacteria including aerobic and anaerobic bacteria was conducted using the feed of Example 1 and the 7-week old mice employed in Example 1.

In the present example, during 23 weeks (161 days) before use of the mice in the confirmation test, the mice of an intestinal bacteria reducing agent administration group was allowed to freely take the feed of Example 1 and water, and a control group (comparative example) was allowed to freely take the normal feed and water. For comparison between the number of intestinal bacteria in a normal animal and the number of intestinal bacteria in an intestinal inflammation model animal, a wild-type animal (mouse) was allowed to freely take normal feed and water.

Fig. 7 shows bar graphs in which the numbers of respective types of intestinal bacteria in feces when mice ingest the feed of the present example (Example 3) are compared with the numbers of respective types of intestinal bacteria in feces when the mice ingest normal feed (comparative example). The results for the wild-type animal are shown as a reference example in Fig. 7. The number of intestinal bacteria was measured in the same manner as in Example 1.

As shown in Fig. 7, the number of bacteria in the comparative example sharply increased after 23 weeks (161 days), regardless of whether aerobic or anaerobic bacteria. On the other hand, it was confirmed that the number of bacteria in the present example decreased significantly or remarkably in more aggressive phraseology as compared to the number of bacteria in the comparative example. On the other hand, it was confirmed that the number of bacteria in the present example decreased to a number comparable to that in a reference example which is the result for the wild-type animal.

It is worth noting that unlike Example 1, the present example showed a significant decrease not only in the number of anaerobic bacteria but also in the number of aerobic bacteria. Therefore, it was confirmed that ingestion of the intestinal bacteria reducing agent enabled reduction of the number of aerobic and anaerobic bacteria which remarkably increases in intestinal inflammation model animals.

### <Confirmation test for actions of bonding, absorbing or capturing intestinal bacteria by intestinal bacteria reducing agent>

### (Example 4)

In the present example, a confirmation test for actions of bonding, absorbing or capturing intestinal bacteria by the intestinal bacteria reducing agent was conducted. Specifically, intestinal bacteria collected from the contents of the cecum of 8-week-old mice were cultured for 24 hours by a static culture method under anaerobic conditions in a culture solution containing the intestinal bacteria reducing agent according to the first embodiment. Specifically, 0.1 g of the intestinal bacteria reducing agent to 2 mL (milliliter) of a bacterial culture solution. Thereafter, a confirmation test for actions of bonding, absorbing or capturing intestinal bacteria by the silicon fine particles was conducted. The intestinal bacteria collected from the contents of the cecum and cultured for 24 hours by a static culture method under anaerobic conditions without adding the intestinal bacteria reducing agent.

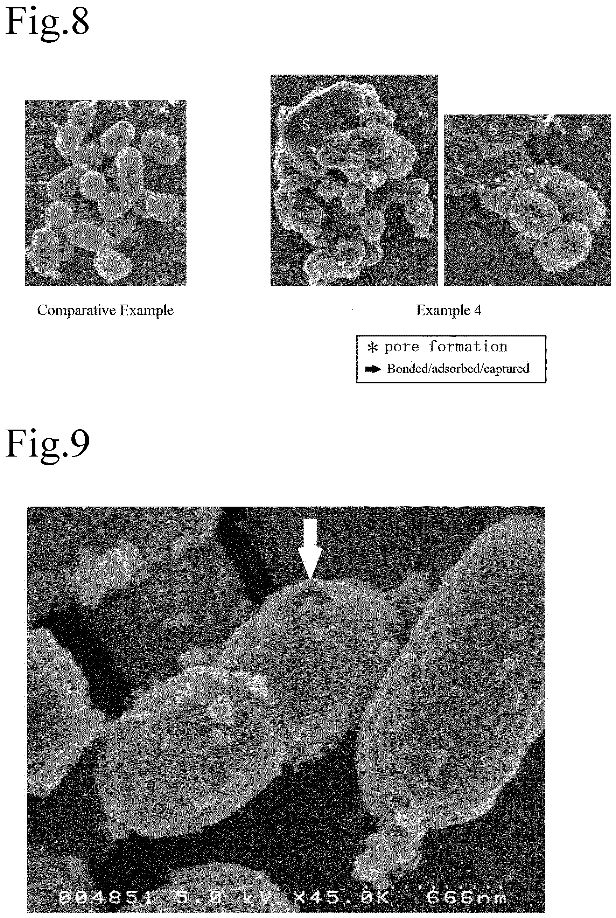

Fig. 8 shows SEM (scanning electron microscope) photographs showing the result of observing silicon fine particles in the intestinal bacteria reducing agent and intestinal bacteria in Example 4 and the result of the comparative example. Fig. 8 shows three SEM photographs which are SEM photographs at 13,000-fold magnification (comparative example), 13,000-fold magnification (Example 4) and 20,000-fold magnification (Example 4) arranged in this order from the left side. Note that "S" in Fig. 8 represents silicon fine particles, and the arrow in Fig. 8 represents a state in which intestinal bacteria are bonded to or adsorbed to (otherwise captured on) the silicon fine particles.

A very interesting fact was confirmed that as shown in Fig. 8, intestinal bacteria were bonded to, adsorbed to, or captured on the surfaces of silicon fine particles directly and/or indirectly with an oxide film interposed therebetween, where the oxide film was formed on the surfaces of the silicon fine particles.

Thus, the fact that the action of bonding, adsorbing or capturing intestinal bacteria, which is an action different from the hydrogen generating capability that can be exhibited by the silicon fine particles, was confirmed indicates that the silicon fine particles have a specific action that cannot be explained by the technical content disclosed in, for example, Non-Patent Document 1.

By closer examination, pore formation was observed in cell membranes in a part of intestinal bacteria in the present example as indicated by the asterisk in Fig. 8. Fig. 9 shows a SEM photograph at 45,000-fold magnification, which shows the result of observing silicon fine particles in the intestinal bacteria reducing agent and intestinal bacteria in the present example. The spots indicated by the arrows in the figure may be pores of the cell membranes. Thus, since an induction phenomenon seen in some antimicrobial peptides was confirmed even when the intestinal bacteria reducing agent according to the first embodiment, which is unrelated to the antimicrobial peptide was used, it is considered that breakage of cell membranes of bacteria by the intestinal bacteria reducing agent can also contribute to the intestinal bacteria reducing effect. Therefore, it was confirmed that the action of adsorbing, bonding or capturing intestinal bacteria by silicon fine particles produced an action of breaking cell membranes.

### <Additional confirmation test for actions of bonding, absorbing or capturing intestinal bacteria by intestinal bacteria reducing agent>

### (Example 5)

In addition to the results of analysis on the action and effect of the intestinal bacteria reducing agent using a bacteria culture solution, which had been confirmed in Example 4, the present inventors confirmed the action and effect of the intestinal bacteria reducing agent within the bodies of 8-week-old mice in Example 4.

Specifically, first, silicon fine particles (intestinal bacteria reducing agent) containing a physiologically acceptable pressure sensitive adhesive, thickener or the like and obtained by passing through the first classification step, the pulverization step and the second classification step in the first embodiment are incorporated in physiological saline at a proportion of 150 mg/mL. Thereafter, a total of 1,600 µL of the mixture was forcibly administered to the mice at substantially equal time intervals and in four equally divided doses.

26 hours after the first administration, i.e., 14 hours after the last administration of the silicon fine particles, the contents of the cecum of the mice were taken out, and the silicon fine particles and intestinal bacteria were then observed with a scanning electron microscope.

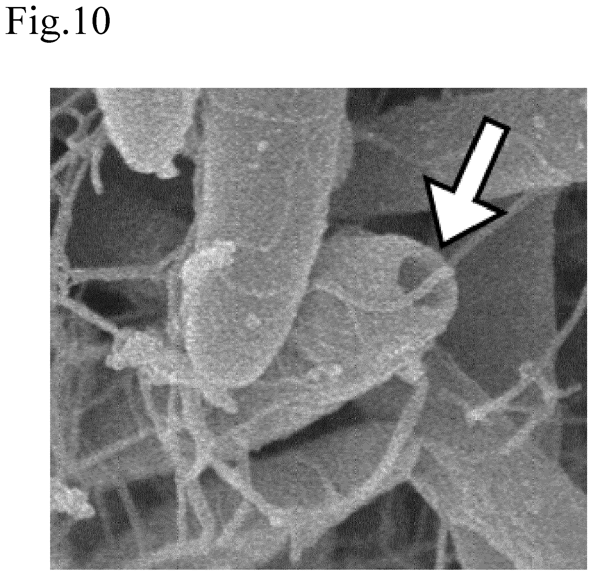

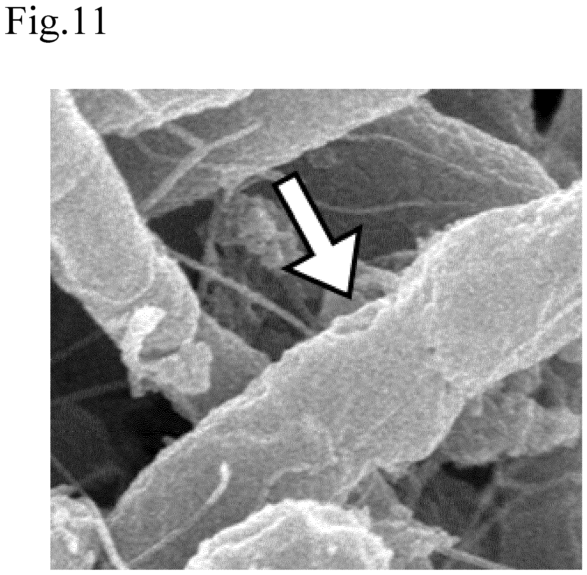

Fig. 10 shows a SEM photograph at 40,000-fold magnification, which shows the result of observing intestinal bacteria in the cecum in the present example. Fig. 11 shows another SEM photograph at 35,000-fold magnification, which shows the result of observing intestinal bacteria in the cecum in the present example.

It was found that a shape appearing to be a pore of a cell membrane was formed in a part of the intestinal bacteria as indicated by the arrows in Figs. 10 and 11. Therefore, it was confirmed again that the silicon fine particles playing a role as an intestinal bacteria reducing agent also caused breakage of the cell membranes of intestinal bacteria within the bodies. Therefore, it was confirmed again that the action of adsorbing, bonding or capturing intestinal bacteria by silicon fine particles produced an action of breaking cell membranes.

### <Other embodiments>

In the intestinal bacteria reducing agent according to the present embodiment, a solid preparation obtained by kneading normal feed, silicon fine particles and citric acid is employed as described above, but the intestinal bacteria reducing agent according to the present embodiment is not limited to such an aspect. For example, a solid preparation obtained using a saccharide or starch instead of the citric acid is another aspect that can be employed in the present embodiment. As an example of another aspect, various known dosage forms that can be ingested by oral ingestion can be appropriately employed depending on the conditions of individuals (living bodies) that ingest the intestinal bacteria reducing agent.

An example of the dosage form is a capsule preparation obtained by filling or incorporating the silicon fine particles according to the present embodiment in a capsule made from a known gelatin component or the like. Use of a known enteric-coated capsule for the capsule preparation is also a preferred aspect. Another example of the dosage form is a film-coated tablet in which silicon fine particles are covered with a known enteric-coated film made from cellulose acetate phthalate, hydroxypropyl methylcellulose phthalate, polyvinyl acetate phthalate or the like, or a known film made from a saccharide. Employment of a known coating agent hardly soluble in the stomach as the film-coated tablet is a preferred aspect. As other examples of the dosage form, granules, powders, syrups (dry syrups), or jellies can be employed.

In the intestinal bacteria reducing agent according to the present embodiment, the proportion of the silicon fine particles mixed when the amount of normal feed is assumed to be 100 is 2.5 in terms of mass ratio, but the proportion of the silicon fine particles mixed is not limited to this numerical value. For example, as described above, when a capsule or a film-coated tablet is employed as the dosage form, the intestinal bacteria reducing agent according to the present embodiment does not require the normal feed.

As described above, the disclosure of each of the embodiments and examples is intended for explanation of the embodiments or the examples, and is not intended to limit the present invention. In addition, claims also include other modifications within the scope of the present invention including other combinations of the embodiments and the examples.

### INDUSTRIAL APPLICABILITY

The intestinal bacteria reducing agent of the present invention can be widely used as at least a part of a drug, food or beverage for regulating the intestinal environment, or a drug for various diseases associated with abnormality of intestinal bacteria.

## Claims

1. An intestinal bacteria reducing agent comprising silicon particles or silicon fine particles, the intestinal bacteria reducing agent being a reducing agent that reduces the number of bacteria in an intestine.

2. The intestinal bacteria reducing agent according to claim 1, wherein the bacteria include anaerobic bacteria.

3. The intestinal bacteria reducing agent according to claim 1, which reduces a ratio of a number of 16S rRNA gene fragments of anaerobic bacteria to a total number of 16S rRNA gene fragments contained in a PCR amplification product obtained by amplifying the 16S rRNA gene using a PCR method.

4. The intestinal bacteria reducing agent according to claim 2 or 3, wherein the anaerobic bacteria are Gram-negative anaerobic bacteria.

5. The intestinal bacteria reducing agent according to any one of claims 2 to 4, wherein the anaerobic bacteria include Bacteroidaceae.

6. A pharmaceutical product for reducing the number of the bacteria in the intestine, the pharmaceutical product comprising the intestinal bacteria reducing agent according to any one of claims 1 to 5.

7. A food or beverage for reducing the number of the bacteria in the intestine, the food or beverage comprising the intestinal bacteria reducing agent according to any one of claims 1 to 5.
